# EUROPEAN PATENT SPECIFICATION

(11) **EP 0 888 054 B1**
(45) Date of publication and mention of the grant of the patent: **03.09.2003**
(21) Application number: 97915020.8
(22) Date of filing: 12.03.1997
(51) Int. Cl.: A01N 43/04, A61K 31/70, C12N 15/63, A61B 8/00, A61K 41/00

(54) **COMPOSITION FOR THE SITE SPECIFIC DELIVERY OF A DRUG AND A METHOD OF USE**
ZUSAMMENSETZUNG ZUR ZIELORTSPEZIFISCHEN VERABREICHUNG EINES MEDIKAMENTES UND VERFAHREN ZUR ANWENDUNG
COMPOSITIONS D'APPORT DE MEDICAMENTS CIBLE SUR UN SITE SPECIFIQUE ET PROCEDE D'UTILISATION

(30) Priority: 12.03.1996 US 615495
(43) Date of publication of application: 07.01.1999
(73) Proprietor: BOARD OF REGENTS OF THE UNIVERSITY OF NEBRASKA, Lincoln, NE 68503 (US)
(72) Inventor: PORTER, Thomas, R., Lincoln, NE 68503 (US); IVERSEN, Patrick, L., Lincoln, NE 68503 (US); MEYER, Gary, D., Lincoln, NE 68503 (US)
(74) Representative: VOSSIUS & PARTNER
(86) International application number: US9703876
(87) International publication number: WO97033474

(56) References cited:
- WO-A-97/14409
- WO-A-98/17324
- US-A- 5 315 998
- PORTER T R ET AL: "Thrombolytic enhancement with perfluorocarbon-exposed sonicated dextrose albumin microbubbles." AMERICAN HEART JOURNAL, (1996 NOV) 132 (5) 964-8. JOURNAL CODE: 3BW. ISSN: 0002-8703., XP000198714 United States
- PORTER T R ET AL: "INTERACTION OF DIAGNOSTIC ULTRASOUND WITH SYNTHETIC OLIGONUCLEOTIDE-LABELED PERFLUOROCARBON-EXPOSED SONICATED DEXTROSE ALBUMIN MICROBUBBLES" JOURNAL OF ULTRASOUND IN MEDICINE, vol. 15, no. 8, August 1996, pages 577-584, XP002054859
- ANTISENSE RESEARCH AND DEVELOPMENT, 1995, Vol. 5, SRINIVASAN et al., "Characterization of Binding Sites, Extent of Binding and Drug Interactions of Oligonucleotides With Albumin", pages 131-139.
- CIRCULATION, 1995, Vol. 92, No. 9, PORTER et al., "Transient Myocardial Contrast After Initial Exposure To Diagnostic Ultrasound Pressures With Minute Doses of Intravenously Injected Microbubbles: Demonstration and Potential Mechanisms", pages 2391-2395.
- INTERNATIONAL JOURNAL OF CARDIAC IMAGING, June 1995, Vol. 11, PORTER et al., "The Mechanism and Clinical Implication of Improved Left Ventricular Videointensity Following Intravenous Injection of Multi-Fold Dilutions of Albumin With Dextrose", pages 117-125.

## Description

This invention relates to a new and improved composition, a pharmaceutical composition comprising said composition and use of the composition for the preparation of a pharmaceutical composition or a diagnostic composition for delivery of bioactive substances. The composition of the invention can be used with several agents and can achieve site specific delivery of a biologically active substance. This can allow for lower doses and for improved efficacy of drugs particularly agents such as oligonucleotides which are plagued with problems in achieving therapeutic concentrations in target organs.

Drug delivery techniques are employed in the formulation of all drug therapy to augment drug availability, to reduce drug dose, and consequently to reduce drug-induced side effects. These techniques serve to control, regulate, and target the release of drugs in the body. The goals have been to provide less frequent drug administration, to maintain constant and continuous therapeutic levels of a drug in the systemic circulation or at a specific target organ site, to achieve a reduction in undesirable side effects, and to promote a reduction in the amount and dose concentration required to realize the desired therapeutic benefit.

To date, drug delivery systems have included drug carriers based upon proteins, polysaccharides, synthetic polymers, erythrocytes, DNA and liposomes. New generation biologicals such as monoclonal antibodies, gene therapy vectors, anti-cancer drugs such as taxol, viral based drugs, and ODN and poly nucleotides have presented several problems with regard to delivery. In fact drug delivery may be the primary hurdle to achieving mainstream therapeutic use of these agents whose initial potential seemed unlimited but whose therapeutic parameters have prevented realization of full benefit.

synthetic oligodeoxyribonucleotides (ODN's) which are chemically modified to confer nuclease resistance represent a fundamentally different approach to drug therapy. The most common applications to date are antisense ODN's with sequences complementary to a specific targeted mRNA sequence. An antisense oligonucleotide approach to therapy involves a remarkably simple and specific drug design concept in which the ODN causes a mechanistic intervention in the processes of translation or an earlier processing event. The advantage of this approach is the potential for gene-specific actions which should be reflected in a relatively low dose and minimal non-targeted side effects.

Phosphorothioate analogs of polynucleotides have chiral internucleoside linkages in which one of the non-bridging ligands is sulfur. The phosphorothioate analog is currently the most commonly employed analogue in biological studies including both *in vitro* and *in vivo.* The most apparent disadvantages of phosphorothioate oligonucleotides include the high cost of preparation of sufficient amounts of high quality material and non-specific binding to proteins. Hence, the primary advantages of the antisense approach (low dose and minimal side effects) fall short of expectations.

Drug delivery efforts with regard to oligonucleotides and polynucleotides have focused on two key challenges; transfection of oligonucleotides into cells and alteration of distribution of oligonucleotides *in vivo*.

Transfection involves the enhancement of *in vitro* cellular uptake. Biological approaches to improve uptake have included viral vectors such as reconstituted viruses and pseudovirions, and chemicals such as liposomes. Methods to improve biodistribution have focused on such things as cationic lipids, which are postulated to increase cellular uptake of drugs due to the positively charged lipid attraction to the negatively charged surfaces of most cells.

Lipofection and DC-cholesterol liposomes have been reported to enhance gene transfer into vascular cells in vivo when administered by catheter. Cationic lipid DNA complexes have also been reported to result in effective gene transfer into mouse lungs after intratracheal administration.

Cationic liposomal delivery of oligonucleotides has also been accomplished, however, altered distribution to the lung and liver was experienced. Asialoglycoprotein poly(L)-lysine complexes have met with limited success as well as complexation with Sendai virus coat protein containing liposomes. Toxicity and biodistribution, however, have remained significant issues.

From the foregoing it can be seen that a targeted drug delivery system for delivery of biologics, particularly poly- and oligonucleotides is needed for these drugs to achieve their fullest potential.

One object of this invention is to provide a novel composition of matter to deliver a pharmaceutical agent to a targeted site in *vivo*.

Another object of the invention is to provide a use of said composition for the preparation of a pharmaceutical composition or diagnostic composition for delivering a pharmaceutical agent to a specific site with the use of diagnostic ultrasound.

Another object of the invention is to provide a composition for enhancing the effectiveness of currently available drugs including plasma-bound drugs with the use of albumin or orosomucoid-albumin perfluorocarbon containing microbubbles when combined with diagnostic ultrasound.

Other objects of the inventions will become apparent from the description of the invention which follows.
According to the invention a new biologically active agent delivery composition and the use thereof are disclosed.
The present invention provides a composition for delivering to a target site a biologically active agent which conjugates with a filmogenic protein, said composition comprising: an aqueous polysaccharide suspension comprising filmogenic protein encapsulated, insoluble gas-filled microbubbles, wherein said insoluble gas is a fluorocarbon gas or SF₆, and wherein said biologically active agent is conjugated to said filmogenic protein.

Furthermore, the present invention provides a pharmaceutical composition or a diagnostic composition comprising the composition.

Also provided by the present invention is a use of the composition as described above for the preparation of a pharmaceutical composition or diagnostic composition for delivering a biologically active agent which conjugates with a filmogenic protein to a specific target tissue site by forming a suspension of filmogenic protein encapsulated, insoluble gas-filled microbubbles, said microbubbles conjugated to said biologically active agent; administering said suspension to an animal; and exposing said tissue site to ultrasound so that said biologically active agent is released at said site.

The compositions can be used to deliver agents such as therapeutics or diagnostics which have been plagued with delivery problems such as oligonucleotides as well as traditional agents and can drastically reduce the effective dosages of each, increasing the therapeutic index and improving bioavailability. This in turn can reduce drug cytotoxicity and side effects. Furthermore, this invention can dramatically enhance the effectiveness of other plasma bound drugs such as heparin, diltiazem, lidocaine, propanolol, cyclosporine, and chemotherapeutic agents which require blood pool activation. For example, we have shown that the anticoagulant properties of heparin can be dramatically enhanced by first combining the medicament with orosomucoid-labeled perfluorocarbon exposed sonicated dextrose albumin, and then given the combination intravenously.

The invention employs equilibrium binding of the biologic agent with a filmogenic protein which is formed as a protein shell microbubble encapsulating an insoluble gas. The conjugates are designed for parenteral administration as an aqueous suspension. After administration and timed with travel of the injected bolus to the site of interest, energy in the form of sound waves cause the microbubbles to cavitate; the agent is then released and concomitantly delivered to the organ or other site or interest.

A Lineweaver-Burke plot of the binding data for PESDA microbubbles with PS-ODN showed that the equilibrium dissociation constant Km (calculated for the 7 concentrations which were run in duplicate) for the binding to the microbubbles was 1.76 X 10⁻⁵M. This is nearly within the range observed for binding a 15mer PS-ODN with sequence 5'd(AACGTTGAGGGGCAT)-3' (SEQ ID NO:1) to human serum albumin in solution of 3.7 - 4.8 X 10-5M, previously reported in Srinivasan SK et al., "Characterization of binding sites, extent of binding, and drug interactions of oligonucleotides with albumin", Antisense Res. Devel. 5:131, 1995.

Ultrasonic imaging has long been used as a diagnostic tool to aid in therapeutic procedures. It is based on the principle that waves of sound energy can be focused upon an area of interest and reflected to produce an image. Generally an ultrasonic transducer is placed on a body surface overlying the area to be imaged and ultrasonic energy, produced by generating and receiving sound waves, is transmitted. The ultrasonic energy is reflected back to the transducer where it is translated into an ultrasonic image. The amount and characteristics of the reflected energy depend upon the acoustic properties of the tissues, and contrast agents which are echogenic are preferably used to create ultrasonic energy in the area of interest and improve the imaging received.

Contrast echocardiography has been used to delineate intracardiac structures, assess valvular competence, and demonstrate intracardiac shunts. Myocardial contrast echocardiography (MCE) has been used to measure coronary blood flow reserve in humans. MCE has been found to be a safe and useful technique for evaluating relative changes in myocardial perfusion and delineating areas at risk.

Ultrasonic vibration has also been used at therapeutic levels in the medical field to increase the absorption of various medicaments. For example, Japanese Patent Kokai number 115591/1977 discloses that percutaneous absorption of a medicament is enhanced by ultrasonic vibration. U.S. Patent Nos. 4,953,565 and 5,007,438 also disclose a technique of percutaneous absorption of medicaments by the aid of ultrasonic vibration. U.S. Patent No. 5,315,998 discloses a booster for drug therapy comprising microbubbles in combination with ultrasonic energy to allow the medicament to diffuse and penetrate. This patent discloses the use of therapeutic levels of ultrasound for up to 20 minutes in contrast to the present invention which uses diagnostic levels of ultrasound with exposure for much shorter time periods to achieve release of conjugated bioactive agents.

Porter *et al.* (*Circulation* 92:2391, 1995) discusses the advantage of pulsed ultrasound over conventional ultrasound in imaging studies using PESDA (perfluorocarbon-exposed sonicated dextrose/albumin) microbubbles, and Porter *et al*. (*Int. J. Cardiac Imag*. 11:117, 1995) describes the effect of various dilutions with dextrose on albumin/dextrose imaging contrast agents.

Srinivasan *et al*. (*Antisense Res. & Dev*. 5:131, 1995) describes a study of the binding of phosphorothioate oligonucleotides to albumin and discusses the effects of albumin binding on drug pharmacokinetics. However, there is no suggestion to conjugate drugs to protein microbubbles for the purpose of drug delivery.

Applicant has demonstrated that traditional diagnostic ultrasound therapy (e.g. including peak negative pressures in the range of 0.1 to 3.5 Megapascals, and transmit frequencies in the range of 1.0 to 40 Megahertz) and contrast agents can be used as a specific targeted delivery application to release therapeutic agents at the exact site of interest. Delivery of the biologic agent is precisely targeted and can release the agent not only at the organ of interest but to the exact site on the organ.

In the presence of ultrasound the microbubbles cavitate and release conjugated bioactive agents and thus delivers them to the target site.

The pharmaceutical composition of the invention comprises a liquid suspension containing microbubbles of an insoluble gas having a diameter of 0.1 to 10 microns. The microbubbles are formed by entrapping microbubbles of a gas into a liquid. The microbubbles are made of various insoluble gases such as fluorocarbons or sulfur hexafluoride gas. The liquid includes any liquid which can form microbubbles. Generally any insoluble gas can be used. It must be gaseous at body temperature and be nontoxic. The gas must also form stable microbubbles of average size of between about .1 and 10 microns in diameter when the pharmaceutical composition is sonicated to form microbubbles. Generally perfluorocarbon gases such as perfluoromethane, perfluoroethane, perfluoropropane, perfluorobutane, and perfluoropentane are preferred. Of these gases, perfluoropropane and perfluorobutane are especially preferred because of their demonstrated safety for intraocular injection in humans. They have been used in human studies for intraocular injections to stabilize retinal detachments (Wong and Thompson, Opthamology 95:609-613). Treatment with intraocular perfluoropropane is considered to be the standard of care for treatment of this disorder. The gases must also have a diffusion coefficient and blood solubility lower than nitrogen or oxygen which diffuse once in the internal atmosphere of the blood vessel.

Other inert gases such as sulfur hexafluoride are also useful in the invention provided they have a diffusion coefficient and blood solubility lower than nitrogen or oxygen. The agent of the invention is formulated in a pharmaceutically effective dosage form for peripheral administration to the host in conjunction with ultrasound therapy. Generally such host is a human host, although other mammalian hosts such as canine or equine can also be subject to this therapy.

The pharmaceutical liquid composition of the invention uses a liquid wherein the gaseous microbubbles are stabilized by a filmogenic protein coating. Suitable proteins include naturally occurring proteins such as albumin, human gamma globulin, human apotransferrin, beta lactose, and urease. The invention preferably employs a naturally occurring protein, but synthetic proteins may also be used. Particularly preferred is human serum albumin.

It is also preferred to use an aqueous solution containing a mixture of a pharmaceutically accepted saccharide e.g., dextrose, in combination with the earlier described protein. In a most preferred embodiment the pharmaceutical liquid composition of the invention is a sonicated mixture of commercially available albumin (human), U.S.P. solution (generally supplied as 5% or 25% by weight sterile aqueous solutions), and commercially available dextrose, U.S.P. for intravenous administration. The mixture is sonicated under ambient conditions, i.e. room air temperature and pressure, and is perfused with an insoluble gas (99.9% by weight) during sonication.

In a most preferred embodiment the preparation of the pharmaceutical liquid composition includes diluting a 2% to 10% by weight solution of human serum albumin, two-fold to eight-fold with 5% to 50% by weight of dextrose. Preferably, a three-fold dilution is used. Exemplary of other saccharide solutions of the invention are aqueous monosaccharide solution (e.g. having the formula C₆H₁₂O₆ such as the hexose sugars, dextrose or fructose or mixtures thereof), aqueous disaccharide solution (e.g. having a formula C₁₂H₂₂O₁₁ such as sucrose, lactose or maltose or mixtures thereof), or aqueous polysaccharide solution (e.g. soluble starches having the formula C₆H₁₀O₅(n) wherein n is a whole number integer between 20 and about 200 such as amylase or dextran or mixtures thereof. The saccharide, however, is not essential to achieve the results of the invention.

The microbubbles are formed by sonication, typically with a sonicating horn. Sonication by ultrasonic energy causes cavitation within the dextrose albumin solution at sites of particulate matter or gas in the fluid. These cavitation sites eventually resonate and produce small microbubbles (about 7 microns in size) which are non-collapsing and stable. In general, sonication conditions which produce concentrations of greater than about 4X10⁸m of between about 5 and about 6 micron microbubbles are preferred. Generally the mixture will be sonicated for about 80 seconds, while being perfused with the insoluble gas.

A second method of preparation includes hand agitating 15 ± 2 ml of sonicated dextrose albumin with 8 ± 2 ml of perfluorocarbon gas prior to sonication. Sonication then proceeds for 80 ± 5 seconds.

A third method of preparation, designed to improve the affinity of the microbubble for many plasma-bound drugs, is to add 5 to 30 milligrams of orosomucoid (alpha-acid glycoprotein) to the albumin-dextrose prior to sonication.

These microbubble sizes are particularly ideal since a microbubble must have a mean diameter of less than 10 microns and greater than 0.1 to be sufficient for transpulmonary passage, and must be stable enough to prevent significant diffusion of gases within the microbubble following intravenous injection and during transit to the target site.

The microbubbles are next incubated with the biologically active agent so that the medicament becomes conjugated with the protein coated microbubble. Quite unexpectedly applicant has demonstrated that filmogenic proteins in the form of microbubbles, as previously used in contrast agents, retain their ability to bind compounds, when the microbubbles are filled with an insoluble gas. This is surprising because traditionally it was thought that in the formation of microbubble contrast agents the protein sphere was comprised of denatured protein. For example see U.S. Patent No. 4,572,203, U.S. Patent No. 4,718,433 and U.S. Patent No. 4,774,958, the contents of each of which are incorporated herein by reference. As demonstrated herein, applicant has shown that when an insoluble gas instead of air is used for the microbubble, much of the sonication energy is absorbed by the gas and the protein retains its binding activity. Thus the protein, such as albumin, can bind to biologically active medicaments to form a microbubble agent conjugation. Air filled microbubbles do not retain their binding capabilities and cannot be used in the method of the invention.

The therapy involves the use of a pharmaceutical composition conjugated to protein microbubbles of a diameter of about 0.1 to 10 microns. The invention uses agents and methods traditionally used in diagnostic ultrasound imaging and as such provides a means for visualization of the therapeutic at its target site for delivery of the therapeutic.

Therapeutic agents useful in the present invention are selected via their ability to bind with the filmogenic protein. For example if the filmogenic protein is albumin, the therapeutic can include oligonucleotides, polynucleotides, or ribozymes all of which can bind with albumin and as such can form a conjugation with the microbubble. A list of drugs which bind to albumin at site 1 which is confirmed to remain intact and thus would be useful in the methods and compositions of the present invention with albumin coated microbubbles follows:

| % Albumin | | |
|---|---|---|
| Drug | Binding | Drug Class |
| Naproxen | 99.7 | NSAID^{⊕} |
| Piroxicam | 99.3 | NSAID^{⊕} |
| Warfarin | 99.0 | Anticoagulant |
| Furosemide | 98.8 | Loop diuretic |
| Phenylbutazone | 96.1 | NSAID⊕ |
| Valproic Acid | 93.0 | Antiepileptic |
| Sulfisoxazole | 91.4 | Sufonimide antibiotic |
| Ceftriaxone | 90-95* | Third Generation cephalosporine antibiotic |
| Miconazole | 90.7-93.1* | Antifungal |
| Phenytoin | 89.0 | Antiepileptic |

| | | |
|---|---|---|
| ⊕Nonsteriodal anti inflammatory drug | | |
| *Represents patient-to-patient variability | | |

If orosomucoid is added to the dextrose albumin, then the list of drugs which could be potentiated would also include erythromycin (antibiotic), lidocaine (antiarrhythmic), meperidine (analgesic), methadone (analgesic), verapamil and diltiazem (antianginals), cyclosporine (an immunosuppresant), propanolol antihypertensive and antianginal), and quinidine (antiarrhythmic).

Other drugs which bind with albumin, particularly at site 1 would also be useful in this embodiment and can be ascertained by those of skill in the art through drug interaction and pharmacology texts standard to the art such as "Drug Information" AHFS 1994 or "Facts and Comparisons" published by Berney Olin updated every quarter. Assays for determination of appropriate protein-biologic agent combinations are disclosed herein and can be used to test any combination for its ability to work with the method of the invention.

According to one embodiment of the invention, albumin coated microbubbles of perfluorocarbon gas have been found to form stable conjugates with oligonucleotides. The ODN conjugated bubbles are then parenterally introduced to the animal as in traditional ultrasound visualization.

A diagnostic ultrasound field is then introduced when the injected bolus reaches the target site. This can be accomplished in any of a number of ways. For example, the ultrasound transducer can be placed directly above the target site. Thus when the injectate reaches this site it will be exposed to the field and the agent will be released. This method will also allow for visualization as the bolus enters the ultrasound field. The energy provided by the ultrasound will cause the microbubble to release the agent at the therapeutic site or into the blood pool. Alternatively, the ultrasound transducer can be placed over a site in the blood pool which will permit constant exposure of the microbubbles as they pass through the circulation. The purpose of this is to enhance the systemic effects of certain drugs, such as heparin. Examples of placement sites where this could occur would be over the heart chambers, aorta, or vena cavae. Another method would be to use the contrast agent alone to time the travel from administration to therapeutic site and then inject the biologic conjugated microbubble suspension with application of ultrasound after the appropriate time interval.

This invention is of particular relevance for oligonucleotide and polynucleotide therapy as the primary hurdle to effective anti-sense, anti-gene, probe diagnostics or even gene therapy employing viral or plasmid nucleotide delivery is the ability of the therapeutic to reach the target site at high enough concentrations to achieve a therapeutic effect.

Therapeutic sites can include such things as the location of a specific tumor, location of a specific infection, an organ which due to differential gene activation expresses a particular gene product, the site of an injury or thrombosis, a site for further processing and distribution of the therapeutic etc.

The method preferred for practicing the delivery therapy of the invention involves obtaining a pharmaceutical liquid agent of the invention, and first introducing said agent into a host by intravenous injection, intravenously (i.v. infusion), percutaneously or intramuscularly. The injectate can then be monitored or timed until the target area is reached by the injectate. Diagnostic ultrasound is introduced at the target site using a suitable Doppler or ultrasound echo apparatus so that the field of ultrasound encompasses the target site and medicament is released from the microbubble. The ultrasound signal activates the microbubbles so that the microbubble-therapeutic bond is broken and the agent is released at the effective area. The time period will generally vary according to the organ of interest, as well as the injection site. It must only be long enough for some of the agent to have reached the organ of interest.

The desired ultrasound is applied by conventional ultrasonic devices which can supply an ultrasonic signal of 20 Khz to several Mhz and is generally applied from about 3 to about 5 Mhz.

Methods of diagnostic ultrasound imaging in which microbubbles, formed by sonicating an aqueous protein solution, are injected into a mammal to alter the acoustic properties of a predetermined area, which is then ultrasonically scanned to obtain an image for use in medical procedures, are well known. For example see U.S. Patent No. 4,572,203, U.S. Patent No. 4,718,433 and U.S. Patent No. 4,774,958.

It is the use of these types of contrast agents as a pharmaceutical composition and application of ultrasound as a targeted delivery system that is the novel improvement of this invention.

The invention has been shown to drastically improve the efficiency and therapeutic activity of several drugs, including most notably antisense oligonucleotides, which have been traditionally plagued with ineffective pharmacologic parameters, including high clearance rate and toxicity.
The protein substance such as human serum albumin is easily metabolized within the body and excreted outside and hence is not harmful to the human body. Further, gas trapped within the microbubbles is extremely small and is easily dissolved in blood fluid; perfluoropropane and perfluorobutane have long been known to be safe in humans. Both have been used in humans for intra ocular injections to stabilize retinal detachments. Wong and Thompson, Ophthalmology 95:609-613. Thus, agents of the invention are extremely safe and nontoxic for patients.

The invention is particularly useful for delivery of nucleotide sequences in the form of gene therapy vectors, diagnostic nucleotide probes, or anti-sense or anti-gene type strategies to ultimately alter or detect gene expression in target cells.

Traditional limitations of oligonucleotide therapy have been preparation of the oligonucleotide analogue which is substantially resistant to the endo-and exonucleases found in the blood and cells of the body. While unmodified ODN's have been shown to be effective, several modifications to these ODN's have helped alleviate this problem.

Modified or related nucleotides of the present invention can include one or more modifications of the nucleic acid bases, sugar moieties, internucleoside phosphate linkages, or combinations of modifications at these sites. The internucleoside phosphate linkages can be phosphorothioate, phosphoroamidite, methylphosphonate, phosphorodithioate and combinations of such similar linkages (to produce mix backbone modified oligonucleotides). Modifications may be internal or at the end(s) of the oligonucleotide molecule and can include additions to the molecule of the internucleoside phosphate linkages, such as cholesterol, diamine compounds with varying numbers of carbon residues between the amino groups, and terminal ribose, deoxyribose and phosphate modifications which cleave, or crosslink to the opposite chains or to associated enzymes or other proteins which bind to the genome. These modifications traditionally help shield the ODN from enzymatic degradation within the cell. Any of the above modifications can be used with the method of the invention. However, in a preferred embodiment the modification is a phosphorothioate oligonucleotide.

Antisense oligonucleotides represent potential tools in research and therapy by virtue of their ability to specifically inhibit synthesis of target proteins. A major theoretical advantage of these ODN's is their potential specificity for binding to one site in the cell. According to one embodiment of the invention a synthetic oligonucleotide of at least 6 nucleotides, preferably complementary to DNA (antigene) or RNA (antisense), which interferes with the process of transcription or translation of endogenous proteins is presented.

Any of the known methods for oligonucleotide synthesis can be used to prepare the oligonucleotides. They are most conveniently prepared using any of the commercially available, automated nucleic acid synthesizers, such as Applied Biosystems, Inc., DNA synthesizer (Model 380B), according to manufacturers protocols using phosphoroamidite chemistry, Applied Biosystems (Foster City, CA). Phosphorothioate oligonucleotides were synthesized and purified according to the methods described in Stek and Zon J. Chromatography, 326:263-280 and in Applied Biosystems, DNA Synthesizer, User Bulletin, Models 380A-380B-381A-391-EP, December 1989. The ODN is introduced to cells by methods which are known to those of skill in the art. See Iversen, et al., "Anti-Cancer Drug Design", 1991, 6531-6538, incorporated herein by reference.

The following examples are for illustration purposes only and are not intended to limit this invention in any way. It will be appreciated by those of skill in the art, that numerous other protein-bioactive agent combinations can be used in the invention and are even contemplated herein. For example, if the filmogenic protein is alpha-one acid glycoprotein, the bioactive agent could be lidocaine, inderal, or heparin.

In all the following examples, all parts and percentages are by weight unless otherwise specified, all dilutions are by volume.

### EXAMPLE 1

### Phosphorothioate oligonucleotide synthesis

Chain extension syntheses were performed on a 1 µmole column support on an ABI Model 391 DNA synthesizer (Perkin Elmer, Foster City, CA) or provided by Lynx Therapeutics, Inc. (Hayward CA). The 1 micromole synthesis employed cyanoethyl phosphoroamidites and sulfurization with tetraethylthiuram disulfide as per ABI user Bulletin 58.

Radiolabeled oligonucleotides were synthesized as hydrogen phosphonate material by Glen Research (Bethesda, MD). The uniformly ³⁵S-labeled PS-ODN with sequences 5'-TAT GCT GTG CCG GGG TCT TCG GGC 3' SEQ ID NO:2 (24-mer complementary to c-myb) and 5' TTAGGG 3' SEQ ID NO:3 were incubated in a final volume of 0.5 ml with the perfluorocarbon-exposed sonicated dextrose albumin microbubble solution for 30 minutes at 37°C. The solutions were allowed to stand so that the bubbles could rise to the top and 100 microliters were removed from the clear solution at the bottom and 100 microliters were removed from the top containing the microbubbles. It is to be understood that the method of the invention is nonsequence specific and any base sequence composition of the ODN can be used.

### Preparation of Microbubble Agent

Five percent human serum albumin and five percent dextrose were obtained from a commercial source. Three parts of 5% dextrose and one part 5% human serum albumin (total 16 milliliters) were drawn into a 35-milliliter Monojet syringe. Each dextrose albumin sample was hand agitated with 8±2 milliliters of either a fluorocarbon gas (decafluorobutane; molecular weight 238 grams/mole) or 8±2 milliliters of room air, and the sample was then exposed to electromechanical sonication at 20 kilohertz for 80±5 seconds. The mean size of four consecutive samples of the perfluorocarbon-exposed sonicated dextrose albumin (PESDA) microbubbles produced in this manner, as measured with hemocytometry was 4.6±0.4 microns, and mean concentration, as measured by a Coulter counter was 1.4x10⁹ bubbles/milliliter. The solution of microbubbles was then washed in a 1000 times volume excess of 5% dextrose to remove albumin which was not associated with the microbubbles. The microbubbles were allowed four hours to rise. The lower solution was then removed leaving the washed foam. The washed foam was then mixed with 0.9% sodium chloride.

### Binding Assays

The radioactive 24-mer PS-ODN was added to a washed solution of PESDA and room air sonicated dextrose albumin (RA-SDA) microbubbles at a concentration of 5nM. Nonradioactive PS-ODN 20-mer was added to tubes containing radioactive 24-mer in a series of increasing concentrations (0, 3.3, 10, 32.7, 94.5, 167, and 626 µM). The suspension of bubbles is mixed by inversion and incubated at 37°C for 60 minutes.

### Measurement of Radioactivity

Radioactivity in solutions was determined by liquid scintillation counting in a liquid scintillation counter (model LSC7500; Beckman Instruments GmbH, Munich, Germany). The sample volume was 100µl to which 5 ml of Hydrocount biodegradable scintillation cocktail was added and mixed. Samples were counted immediately after each experiment and then again 24 hours later in order to reduce the influence of chemiluminescence and of quenching.

### Flow cytometry

The uniformity of room air versus perfluorocarbon-containing sonicated dextrose albumin microbubble binding of PS-ODN was determined by flow cytometry. A solution of microbubbles was washed in a 1000 fold excess volume of sterile saline. Three groups of samples were prepared in triplicate as follows; Group A (control) in which 100 µl of microbubbles were added to 900 µL of saline, Group B in which 100 µL of microbubbles were added to 900µL of saline and 2µL of FITC-labeled 20-mer was added (final 20-mer concentration is 151 nM), and group C in which 100 µL of microbubbles were added to 800 µL of saline, 2µL of FITC-labeled 20-mer and 100 µL of unlabeled 20-mer(final concentration is 151nM). The incubations were all conducted for 20 minutes at room temperature.

Washed microbubble suspensions were diluted in sterile saline (Baxter) and then incubated with FITC-labeled PS-ODN. Flow cytometric analysis was performed using a FACStar Plus (Becton Dickinson) equipped with 100 mW air-cooled argon laser and the Lysis II acquisition and analysis software. List mode data were employed for a minimum of 10⁴ collected microbubbles and independent analysis a for each sample.

### Study Protocol

A variable flow microsphere scanning chamber was developed for the study which is similar to that we have described previously Mor-Avi V., et al "Stability of albunex microspheres under ultrasonic irradiation; and in vitro study. J Am Soc Echocardiogr 7:S29, 1994. This system consists of a circular scanning chamber connected to a Masterflex flow system(Microgon, Inc., Laguna Hills California) The scanning chamber was enclosed on each side by water-filled chambers and bound on each side by acoustically transparent material. The PS-ODN-labeled PESDA microbubbles (0.1 milliliters) were injected as a bolus over one second proximal to the scanning chamber which then flowed through plastic tubing into a tap water-filled scanning chamber at a controlled flow rate of 100 ml/min. As the bubbles passed through the scanning chamber, the scanner(2.0 Megahertz) frequency, 1.2 Megapascals peak negative pressure) was set to either deliver ultrasound at a conventional 30 Hertz frame rate or was shut off. Following passage through the scanning chamber, the solution was then passed through the same size plastic tubing into a graduated cylinder. The first 10 milliliters was discarded. Following this, the next 10 milliliters was allowed to enter into a collection tube. The collection tube containing the effluent microbubbles was allowed to stand in order to separate microbubbles on the top from whatever free oligonucleotide existed in the lower portion of the sample. Drops from both the upper and lower operation of the effluent were then placed upon a hemocytometer slide and analyzed using a 10X magnification. Photographs of these slides were then made and the number of microbubbles over a 36 square centimeter field were then hand-counted. The upper and lower layers of the remaining effluent were then used for analysis of oligonucleotide content using flow cytometry in the same manner described below.

Microbubble samples exposed to the various oligonucleotide solution were mixed 1:5(v/v) with a solution of formamide and EDTA and heated to 95°C for 5 minutes. These samples were then examined on an Applied Biosystems Model 373A DNA sequencer with 20% polyacrylamide gel. The data were acquired with GeneScanner software so that fluorescence intensity area under the curve could be determined.

The in vivo studies of the effect of these same diagnostic ultrasound pressures on kidney uptake of PS-ODN were performed in three dogs. Intravenous injections of fluorescent PS-ODN labeled PESDA microbubbles (0.2 ml) were given in the femoral vein in these dogs. The left kidney in each dog was being insonified by an externally placed 2.0-2.5 Megahertz diagnostic ultrasound transducer(peak negative pressure 1.1MPa). The kidney was insonified using a 30 Hertz frame rate for a minimum of 2 minutes after injection, and during the visually evident appearance of contrast in the kidney. In each dog, left ventricular and pulmonary artery pressures were monitored before and after kidney injection using saline filled catheters placed in the left ventricle and pulmonary artery, respectively. Approximately four hours after injection, the dog was sacrificed and both kidneys removed. Cut sections were then obtained from the renal cortex and sampled for PS-ODN counted using the gene scanning methods described above. Histologic sections were also obtained for the analysis of fluorescence in the glomeruli and nephrons.

### Analysis of data

Statistical analysis of data were obtained using InStat software (GraphPad, San Diego CA) with both unpaired t-test for comparison of multiple samples and paired t-tests for comparison of partitioning of PS-ODN from the top to bottom of a sample. Graphical analysis of data were obtained using Prism software (GraphPad, San Diego, CA)

### Phosphororthioate Oligonucleotide Binding of PESDA versus RA-SDA Microbubbles

The partitioning of PS-ODN to PESDA microbubbles (top layer) and non-bubble washed (albumin-free) and unwashed (non-bubble albumin containing) lower layers as counted by liquid scintillation counting are demonstrated in Table 1.

**TABLE 1:**

| **OLIGONUCLEOTIDES BIND TO ALBUMIN OF PESDA MICROBUBBLES** | | | | |
|---|---|---|---|---|
| **BUBBLES IN THE PRESENCE OF FREE ALBUMIN** | | | | |
| | N | TOP cpm/µl | BOTTOM cpm/µl | RATIO T/B |
| TTAGGG | 6 | 125±6.4 | 92.3±6.4 | 1.35 |
| c-myb 6 | | 94.1±17.6 | 77.3±1.2 | 1.35 |

| **WASHED BUBBLES (NO FREE ALBUMIN)** | | | | |
|---|---|---|---|---|
| | N | TOP cpm/µl | BOTTOM cpm/µl | RATIO T/B |
| TTAGGG | 6 | 210±10.8 | 126±8.7 | 1.67 |
| c-myb 6 | | 200.3±37.4 | 92.7±15.7 | 2.16 |

These data indicate that albumin in the unwashed solution which is not associated with the microbubble will bind to the PS-ODN so that the partitioning of PS-ODN is equivalent between microbubbles(top layer) and the surrounding solution (lower layer; p=HS). Removal of non microbubble associated albumin (Washed Bubbles in Table 1) does not show a significant partitioning of the PS-ODNs with the PESDA microbubbles (1.67 for TTAGGG PS-ODN and 2.16 for c-myb PS-ODN). The recovery of total radioactivity in the experiments reported in Table 1 is 96% of the radioactivity added which is not significantly different from 100%.

The affinity of binding of PS-ODN to washed microbubbles was evaluated by addition of increasing amounts of excess non-radioactive PS=ODN as a competing ligand for binding sites. In this case a 20mer PS-ODN with sequence 5'-d(CCC TGC TCC CCC CTG GCT CC)-3' (SEQ ID NO: 4) was employed to displace the radioactive 24mer. Albumin protein concentration in the washed microbubble experiments was 0.28 ± 0.04 mg/ml as determined by the Bradford Assay (Bradford M et al., "A Rapid and Sensitive Method for the quantification of microgram quantities of protein utilizing the principle of protein-dye binding", Anal. Biochem. 72:248, 1976). The observed binding data were presented as a Lineweaver Burke plot. The equilibrium dissociation constant Kₘ (calculated for the 7 concentrations which were run in duplicate) for the binding to the microbubbles was 1.76 X 10⁻⁵ M.

The distribution of FITC-labeled microbubbles is provided in Table 2.

**TABLE 2:**

| **DISTRIBUTION OF OLIGONUCLEOTIDE (PS-ODN) BOUND MICROBUBBLES** | | | | | | |
|---|---|---|---|---|---|---|
| | **Control PS-ODN** | | **151nM FITC PS-ODN** | | **Excess Unlabeled ODN** | |
| No. | PE | MI | PE | MI | PE | MI |
| 1 | 99.5 | 2.38 | 98.9 | 2109.8 | 97.8 | 1753.1 |
| 2 | 99.3 | 4.07 | 99.1 | 2142.3 | 98.7 | 1710.9 |
| 3 | 99.4 | 3.52 | 99.1 | 2258.5 | 99.3 | 1832.2 |
| | | | | | | |
| mean | | 3.23 | | 2170 | | 1765 |
| ±SE | | ±0.50 | | ±46¹ | | ±36^{1,2} |

| | | | | | | |
|---|---|---|---|---|---|---|
| PE=percent events | | | | | | |
| MI=mean intensity | | | | | | |
| SE=standard error | | | | | | |
| ¹indicates this mean is significantly different form control, P<0.001 | | | | | | |
| ²indicates this mean is significantly different form 151nM, P<0.001 | | | | | | |

The significant decrease in mean fluorescence intensity in the samples containing excess unlabeled PS-ODN indicates the binding to microbubbles is saturable.. Consequently, since the binding is saturable, the nonspecific interactions of PS-ODN with the microbubble surface are limited. The Gaussian distribution of PS-ODN to washed PESDA microbubbles indicated that the albumin on these microbubbles retained its binding site for the oligonucleotide. The absence of a Gaussian distribution for washed RA-SDA indicated that loss of albumin binding site 1 for this oligonucleotide occurred during sonication of these microbubbles. For a discussion of albumin binding characteristics particularly as they relate to oligonucleotides see Kumar, Shashi et al "Characterization of Binding Sites, Extent of Binding, and Drug Interactions of Oligonucleotides with Albumin" Antisense Research and Development 5: 131-139 (1995) the disclosure of which is hereby incorporated by reference.

### Influence of Diagnostic ultrasound on Ps-ODN Bound Microbubbles; In vitro and In vivo observations

The observed influence of the 2.0 Megahertz diagnostic ultrasound of PS-ODN-labeled PESDA microbubbles in the suspension is provided in Table 3.

**TABLE 3:**

| **ANALYSIS OF PS-ODN AND MICROBUBBLE COUNTS AFTER DIAGNOSTIC ULTRASOUND EXPOSURE TO MICROBUBBLES. Microbubble counts are in parentheses.** | | | | | | |
|---|---|---|---|---|---|---|
| PS-ODN concentration (microbubble Counts) no ultrasound | | | | PS-ODN concentration (microbubble Counts) with ultrasound | | |
| **ODN nM** | **top** | **bottom** | **T/B** | **top** | **bottom** | **T/B** |
| 0.015 | 189 | 158 | 1.2 | 188 | 187 | 1.0 |
| | (112) | (16) | (7.0) | (56) | (12) | (4.7) |
| | | | | | | |
| 0.1 | 169 | 94 | 1.8 | 184 | 191 | 1.0 |
| | (256) | (8.0) | (8.0) | (16) | (32) | (2.0) |
| | | | | | | |
| 1.0 | 209 | 198 | 1.1 | 254 | 255 | 1.0 |
| | (288) | (8.0) | (36.0) | (88) | (40) | (2.0) |
| | | | | | | |
| **Mean** | 203±19 | 147±25 | 1.5±0.2 | 210±18.6 | 219±20.0 | 1.0±0.0 |
| **± SE** | (219±54) | (19±7) | (17±10) | (53±21) | (20±10) | (3±1.0) |
| top= top layer of effluent solution after insonification; | | | | | | |
| bottom = bottom layer of effluent solution after insonification | | | | | | |

There is a significant loss of microbubbles in the top layer following exposure to ultrasound (219±54 microbubbles without ultrasound versus 53±21 microbubbles in the top layer of the effluent when exposed to diagnostic ultrasound). This loss of microbubble counts was evident regardless of the PS-ODN concentration attached to the upper bubble containing layer when no ultrasound was applied. In the presence of diagnostic ultrasound, however, the PS-ODN concentration measured by gel electrophoresis in the lower non-bubble containing layer was significantly increased.

In one dog, there was over a 10-fold greater uptake of PS-ODN in the insonified kidney compared to the noninsonified kidney following intravenous PS-ODN in labeled PESDA. In two of the three dogs, this partitioning of PS-ODN uptake to the insonified kidney was evident. In the first dog, there was a 3-fold higher uptake of PS-ODN in the insonified kidney versus the noninsonified kidney. In the second dog, there was over a nine-fold higher uptake of PS-ODN in the insonified kidney. In dog three, however, there was no difference in PS-ODN uptake between the two kidneys. There were no hemodynamic changes following the intravenous injections of PS-ODN-labeled PESDA microbubbles. Histologic examination of the kidneys post-mortem also indicated no destruction of any glomerular or tubular cells by the diagnostic ultrasound.

Two important aspects of the invention are herein demonstrated. First, PS-ODN binds to the albumin in PESDA microbubbles, indicating that the binding site 1 on albumin is biologically active following production of these bubbles by electromechanical sonication. This binding site affinity is lost when the electromechanical sonication is performed only with room air. Further, removal of albumin not associated with PESDA microbubbles by washing shows a significant partitioning of the PS-ODNs with the microbubbles(Table 1). These observations demonstrate that albumin denaturation does not occur with perfluorocarbon-containing dextrose albumin solutions during sonication as has been suggested with sonication in the presence of air. The retained bioactivity of albumin(especially at site 1) in PESDA microbubbles was confirmed by the affinity of binding of PS-ODN to washed PESDA microbubbles in the presence of increasing amounts of excess non-radioactive PS-ODN as a competing ligand for binding sites (Table 2). The significant decrease in mean fluorescence intensity in the samples containing excess unlabeled PS-ODN indicates the binding to microbubbles is saturable.

Second, exposure of PS-ODN-labeled PESDA microbubbles to diagnostic ultrasound does not alter the integrity of the PS-ODN but does release it from its albumin binding (Table 3).

### EXAMPLE 2

In one dog, intravenous heparin in a bolus dose of 1500 units was given in three different settings. Baseline measurements of activated partial thromboplastin time (PTT) were measured and then at five minute intervals after each injection for a minimum of 15 minutes. In setting 1, the heparin was given as the free drug. In setting 2, 1500 units heparin was given bound to orosomucoid PESDA but NO ultrasound was applied to the blood pool. In setting 3, the same dose of heparin (1500 units) bound to orosomucoid PESDA but ultrasound was applied to the blood pool. In setting 1, the PTT increased to >106 seconds at five minutes, but returned to between 30 to 60 seconds at 15 minutes. In setting 2, the PTT did not reach >106 seconds until 10 minutes, but at 15 minutes it was back to between 60 and 80 seconds. In setting 3, the PTT remained >106 seconds even at 15 minutes. No further measurements were made.

## Claims

1. A composition for delivery of a biologically active agent which conjugates with a filmogenic protein to a target site comprising: an aqueous polysaccharide suspension comprising filmogenic protein-encapsulated, insoluble gas-filled microbubbles and a biologically active agent conjugated to said protein.

2. The composition of claim 1 wherein said protein is selected from the group consisting of albumin, human gammaglobulin, human apotransferrin, beta lactose and urease.

3. The composition of claim 1 or 2 wherein said biologically active agent is selected from the group consisting of an oligonucleotide, a polynucleotide, ribozyme, naproxen, piroxicam, warfarin, furosemide, phenylbutazone, valproic, acid, sulfisoxizole, ceftriaxone, and miconazole.

4. The composition of claim 3 wherein said oligonucleotide is a phosphorothioate oligonucleotide.

5. The composition of claim 4 wherein said phosphorothioate oligonucleotide is an antisense oligonucleotide.

6. The composition of claim 1 or 2 wherein said biologically active agent is a nucleic acid biological agent selected from the group consisting of an antisense oligonucleotide, antigene oligonucleotide, oligonucleotide probe, or a nucleotide vector, viral vector or plasmid.

7. A composition for delivery of a nucleic acid biologically active agent to a target site comprising albumin -encapsulated, insoluble gas-filled microbubbles and a nucleic acid biological agent conjugated to said albumin microbubbles.

8. The composition of any one of claims 1 to 7, wherein said gas is a perfluorocarbon gas.

9. The composition of claim 8, wherein said gas is selected from the group consisting of perfluoromethane, perfluoroethane, perfluoropropane, perfluorobutane, and perfluoropentane.

10. The composition of claim 1, wherein said protein is albumin and said gas is perfluorobutan or perfluoropentane.

11. The composition of claim 10, wherein said protein is albumin, said gas is perfluoropentane and said biologically active agent is as defined in claim 3.

12. The composition of any one of claims 1 to 11, wherein said microbubbles are 0.1 to 10 microns in diameter.

13. The composition of claim 2, wherein said microbubbles are obtainable by the steps of: mixing an aqueous solution comprising 2% to 10% by weight of human serum albumin diluted two-fold to eight-fold with 5% to 50% by weight dextrose; and exposing said solution to a sonication horn to create cavitation at particulate sites in said solution thereby generating stable microbubbles from about 0.1 to 10 microns in diameter.

14. The. composition of claim 13, wherein said dilution of albumin with dextrose is a three-fold dilution.

15. The composition of claim 13 or 14, wherein said human serum albumin is a 5% by weight solution.

16. The composition of any one of claims 13 to 15, wherein said dextrose is a 5% by weight solution.

17. A pharmaceutical composition or a diagnostic composition comprising the composition of any one of claims 1 to 16.

18. The use of the composition of any one of claims 1 to 16 for the preparation of a pharmaceutical composition or diagnostic composition for delivering a biologically active agent which conjugates with a filmogenic protein to specific target tissue sites by forming a solution of filmogenic protein-encapsulated, insoluble gas-filled microbubbles, said microbubbles conjugated to said biologically active agent; administering said solution to an animal; and exposing said tissue site to ultrasound so that said biologically active agent is re!eased at said site.

19. A method for the formation of the microbubbles as defined in claim 2 whereby the method is as defined in any one of claims 13 to 16.

## Patentansprüche

1. Zusammensetzung zum Transport eines biologisch aktiven Wirkstoffs, der mit einem filmogenen Protein konjugiert ist, zu einem Zielort, umfassend: eine wässrige Polysaccharidsuspension umfassend mit einem filmogenen Protein umhüllte, mit einem unlöslichen Gas gefüllte Mikroblasen und einen mit dem Protein konjugierten biologisch aktiven Wirkstoff.

2. Zusammensetzung nach Anspruch 1, wobei das Protein aus der Gruppe bestehend aus Albumin, menschlichem Gammaglobulin, menschlichem Apotransferrin, β-Lactose und Urease ausgewählt ist.

3. Zusammensetzung nach Anspruch 1 oder 2, wobei der biologisch aktive Wirkstoff ausgewählt ist aus der Gruppe bestehend aus einem Oligonucleotid, einem Polynucleotid, Ribozym, Naproxen, Piroxicam, Warfarin, Furosemid, Phenylbutazon, Valproinsäure, Sulfisoxizol, Ceftriaxon und Miconazol.

4. Zusammensetzung nach Anspruch 3, wobei das Oligonucleotid ein Phosphorthioat-Oligonucleotid ist.

5. Zusammensetzung nach Anspruch 4, wobei das Phophorthioat-Oligonucleotid ein Antisense-Oligonucleotid ist.

6. Zusammensetzung nach Anspruch 1 oder 2, wobei der biologisch aktive Wirkstoff ein biologischer Nucleinsäurewirkstoff ist, ausgewählt aus der Gruppe bestehend aus einem Antisense-Oligonucleotid, einem Antigen-Oligonucleotid, einer Oligonucleotidsonde, oder einem Nucleotidvektor, einem viralen Vektor oder Plasmid.

7. Zusammensetzung zum Transport eines biologisch aktiven Nucleinsäurewirkstoffes an einen Zielort, umfassend mit Albumin umhüllte, mit einem unlöslichen Gas gefüllte Mikroblasen und einen mit den Albumin-Mikroblasen konjugierten biologischen Nucleinsäurewirkstoff.

8. Zusammensetzung nach einem der Ansprüche 1 bis 7, wobei das Gas ein Perfluorcarbongas ist.

9. Zusammensetzung nach Anspruch 8, wobei das Gas aus der Gruppe bestehend aus Perfluormethan, Perfluorethan, Perfluorpropan, Perfluorbutan und Perfluorpentan ausgewählt wird.

10. Zusammensetzung nach Anspruch 1, wobei das Protein Albumin und das Gas Perfluorbutan oder Perfluorpentan ist.

11. Zusammensetzung nach Anspruch 10, wobei das Protein Albumin und das Gas Perfluorpentan ist und der biologisch aktive Wirkstoff wie in Anspruch 3 definiert ist.

12. Zusammensetzung nach einem der Ansprüche 1 bis 11, wobei die Mikroblasen einen Durchmesser von 0,1 bis 10 Micron aufweisen.

13. Zusammensetzung nach Anspruch 2, wobei die Mikroblasen erhältlich sind durch die Schritte: Mischen einer wässrigen Lösung umfassend 2 bis 10 Gew.-% menschliches Serumalbumin, das zwei- bis achtfach mit 5 bis 50 Gew.-% Dextrose verdünnt ist; und Aussetzen der Lösung einem Beschallungstrichter, um an bestimmten Stellen in der Lösung Hohlraumbildung und dadurch stabile Mikroblasen mit einem Durchmesser von etwa 0,1 bis 10 Mikron zu erzeugen.

14. Zusammensetzung nach Anspruch 13, wobei die Verdünnung des Albumins mit Dextrose eine dreifache Verdünnung ist.

15. Zusammensetzung nach Anspruch 13 oder 14, wobei das menschliche Serumalbumin eine 5 Gew.-%-Lösung ist.

16. Zusammensetzung nach einem der Ansprüche 13 bis 15, wobei die Dextrose eine 5 Gew.-%-Lösung ist.

17. Arzneimittel oder Diagnosemittel umfassend die Zusammensetzung nach einem der Ansprüche 1 bis 16.

18. Verwendung der Zusammensetzung nach einem der Ansprüche 1 bis 16 zur Herstellung eines Arzneimittels oder eines Diagnosemittels zum Transport eines biologisch aktiven Wirkstoffs, der mit einem filmogenen Protein konjugiert ist, zu bestimmten Zielgewebeorten durch die Bildung einer Lösung aus mit einem filmogenen Protein umhüllten, mit einem unlöslichen Gas gefüllten Mikroblasen, wobei die Mikroblasen mit dem biologisch aktiven Wirkstoff konjugiert sind; die Verabreichung der Lösung an ein Tier; und das Aussetzen des Gewebeorts einem Ultraschall, so dass der biologisch aktive Wirkstoff an dem Ort freigesetzt wird.

19. Verfahren zur Erzeugung der wie in Anspruch 2 definierten Mikroblasen, wobei das Verfahren wie in einem der Ansprüche 13 bis 16 definiert ist.

## Revendications

1. Composition destinée au transport, à un site cible, d'un agent biologiquement actif qui se conjugue à une protéine filmogène, comprenant : une suspension aqueuse de polysaccharide comprenant des microbulles remplies de gaz insoluble encapsulées dans un protéine filmogène, et un agent biologiquement actif conjugué à la dite protéine.

2. Composition selon la revendication 1 dans laquelle ladite protéine est sélectionnée parmi le groupe constitué de l'albumine, de la gammaglobuline humaine, de l'apotransferrine humaine, du bêta lactose et de l'uréase.

3. Composition selon la revendication 1 ou 2 dans laquelle ledit agent biologiquement actif est sélectionné parmi le groupe constitué d'un oligonucléotide, d'un polynucléotide, d'un ribozyme, du naproxène, du piroxicam, de la warfarine, du furosémide, de la phénylbutazone, de l'acide valproïque, du sulfisoxizole, de la ceftriaxone et du miconazole.

4. Composition selon la revendication 3 dans laquelle ledit oligonucléotide est un oligonucléotide phosphorothioate.

5. Composition selon la revendication 4 dans laquelle ledit oligonucléotide phosphorothioate est un oligonucléotide antisens.

6. Composition selon la revendication 1 ou 2 dans laquelle ledit agent biologiquement actif est un agent biologique de type acide nucléique sélectionné parmi le groupe constitué d'un oligonucléotide antisens, un oligonucléotide antigène, une sonde oligonucléotidique, ou un vecteur nucleotidique, un vecteur viral ou un plasmide.

7. Composition destinée au transport d'un agent nucléique biologiquement actif à un site cible comprenant des microbulles remplies de gaz insoluble encapsulées dans de l'albumine et un agent biologique de type acide nucléique conjugué auxdites microbulles d'albumine.

8. Composition selon l'une des revendications 1 à 7, dans laquelle ledit gaz est un gaz perfluorocarboné.

9. Composition selon la revendication 8, dans laquelle ledit gaz est sélectionné parmi le groupe constitué du perfluorométhane, du perfluoroéthane, du perfluoropropane, du perfluorobutane, et du perfluoropentane.

10. Composition selon la revendication 1 dans laquelle ladite protéine est l'albumine et ledit gaz est un perfluorobutane ou un perfluoropentane.

11. Composition selon la revendication 10 dans laquelle ladite protéine est l'albumine, ledit gaz est le perfluoropentane et ledit agent biologiquement actif est tel que défini dans la revendication 3.

12. Composition selon l'une des revendications 1 à 11 dans laquelle lesdites microbulles ont un diamètre de 0,1 à 10 microns.

13. Composition selon la revendication 2, dans laquelle lesdites microbulles sont susceptibles d'être obtenues par les étapes de : mélange d'une solution aqueuse comprenant 2 % à 10 % en poids d'albumine sérique humaine diluée deux à huit fois avec 5 % à 50 % en poids de dextrose ; et exposition de ladite solution à une corne de sonication pour créer une cavitation en des sites particulaires dans ladite solution, générant ainsi des microbulles stables d'environ 0,1 à 10 microns de diamètre.

14. Composition selon la revendication 13 dans laquelle ladite dilution d'albumine avec le dextrose est une dilution par un facteur trois.

15. Composition selon la revendication 13 ou 14, dans laquelle ladite albumine sérique humaine est une solution à 5 % en poids.

16. Composition selon l'une des revendications 13 à 15, dans laquelle ledit dextrose est une solution à 5 % en poids.

17. Composition pharmaceutique ou composition diagnostique comprenant une composition selon l'une des revendications 1 à 16.

18. Utilisation d'une composition selon l'une des revendications 1 à 16 pour la préparation d'une composition pharmaceutique ou d'une composition diagnostique destinée au transport à un site cible d'un agent biologiquement actif qui se conjugue à une protéine filmogène en formant une solution de microbulles remplies de gaz insoluble encapsulées dans une protéine filmogène, lesdites microbulles étant conjuguées audit agent biologiquement actif ; en administrant ladite solution à un animal ; et en exposant ledit site tissulaire à des ultrasons de manière à délivrer ledit agent biologiquement actif audit site.

19. Méthode de formation de microbulles telles que définies à la revendication 2, la méthode étant telle que définie à l'une quelconque des revendications 13 à 16.
